# EUROPEAN PATENT APPLICATION

(11) **EP 3 912 503 A1**
(43) Date of publication of application: **24.11.2021**
(21) Application number: 20741461.6
(22) Date of filing: 15.01.2020
(51) Int. Cl.: A41G 3/00, A41G 5/00, G01K 1/02, G01K 1/14, G01K 7/00

(54) **WIG, INFORMATION PROCESSING DEVICE, HEAD MEASUREMENT METHOD, INFORMATION PROCESSING METHOD, AND PROGRAM**

(30) Priority: 15.01.2019 JP 2019004544
(71) Applicant: Aderans Company Limited, Shinjuku-ku Tokyo 160-8429 (JP)
(72) Inventor: ITO, Toshio, Tokyo 160-8429 (JP); KOSUGE, Toshihiko, Tokyo 160-8429 (JP)
(74) Representative: IP Trust
(86) International application number: PCT/JP2020/000988
(87) International publication number: WO 2020/149286

(57) **Abstract**

This invention provides a wig capable of obtaining data indicative of a head environment. This invention includes at least one sensor (2) and a storage section (31) in which sensed data obtained by the at least one sensor (2) is stored.

## Description

### Technical Field

The present invention relates to a wig, particularly to a wig provided with a sensor for collecting data.

### Background Art

As a conventional technique, there has been known a deodorizing and drying tool which is for wigs and which is provided with a humidity sensor (Patent Literature 1). Citation List

[Patent Literature 1] Japanese Patent No. 3813899 (Publication date: November 6, 2003)

### Summary of Invention

### Technical Problem

Meanwhile, users have potentially sought an appropriate advice in accordance with his/her head environment.

However, the conventional techniques such as the technique described above are not configured to obtain data indicative of a head environment, and therefore cannot provide an appropriate advice in accordance with the data, disadvantageously.

An aspect of the present invention has an object to provide a wig capable of obtaining data indicative of a head environment.

### Solution to Problem

In order to attain the above object, a wig in accordance with an aspect of the present invention includes: at least one sensor; and a storage section in which sensed data obtained by the at least one sensor is stored.

With the above configuration, it is possible to store, in the storage section provided to the wig, data indicative of a head environment obtained by the at least one sensor mounted on the wig.

In order to attain the above object, an information processing device in accordance with an aspect of the present invention includes: an obtaining section configured to obtain sensed data obtained by at least one sensor mounted on a wig; an advice generating section configured to generate, with reference to the sensed data obtained by the obtaining section, advice information indicating an advice for a user; and a display section configured to display the advice information generated by the advice generating section.

With the above configuration, it is possible to obtain the sensed data regarding the head wearing the wig, and to generate an advice in accordance with the obtained sensed data and display the advice thus generated.

In order to attain the above object, a head measurement method in accordance with an aspect of the present invention includes the steps of: obtaining sensed data by carrying out, with use of at least one sensor mounted on the wig, measurement on a head wearing a wig; and storing, in a storage section mounted on the wig, the sensed data thus obtained.

With the above configuration, it is possible to store, in the storage section provided to the wig, data indicative of a head environment obtained by the at least one sensor mounted on the wig.

In order to attain the above object, an information processing method in accordance with an aspect of the present invention includes the steps of: obtaining sensed data obtained by at least one sensor mounted on a wig; generating, with reference to the sensed data thus obtained, advice information indicating an advice for a user; and displaying the advice information thus generated.

With the above configuration, it is possible to obtain the sensed data regarding the head wearing the wig, and to generate an advice in accordance with the obtained sensed data and display the advice thus generated.

### Advantageous Effects of Invention

In accordance with an aspect of the present invention, it is possible to provide a wig capable of obtaining data indicative of a head environment.

### Description of Embodiments

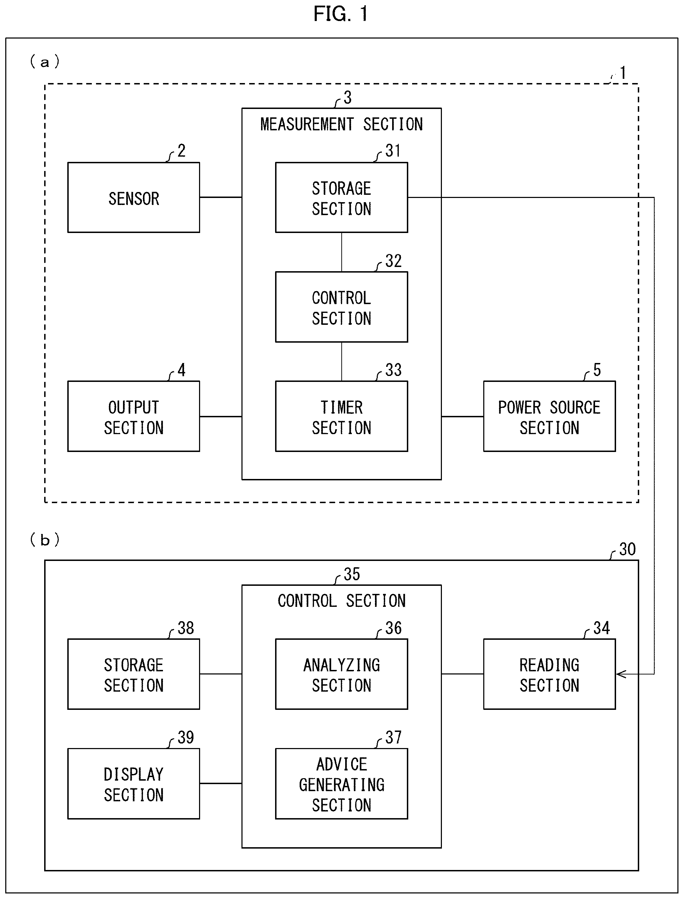

(a) of Fig. 1 is a view schematically illustrating a sensor system mounted on a wig in accordance with Embodiment 1 of the present invention. (b) of Fig. 1 is a view schematically illustrating an advice generating device 30 in accordance with Embodiment 1 of the present invention.
Fig. 2 is a flowchart illustrating a method in accordance with Embodiment 1 of the present invention for generating an advice.
Fig. 3 is a side view schematically illustrating one example of a situation in which a wig base 20 for a full head wig is worn by a user.
Fig. 4 is a back view schematically illustrating the wig base 20 for the full head wig.
Fig. 5 is a top view schematically illustrating one example of a situation in which the wig base 20 for the full head wig is worn by the user.
Fig. 6 illustrates one example of management information generated by the advice generating device 30.
Fig. 7 illustrates one example of the management information generated by the advice generating device 30.
Fig. 8 illustrates one example of the management information generated by the advice generating device 30.
Fig. 9 illustrates one example of the management information generated by the advice generating device 30.
Fig. 10 illustrates one example of the management information generated by the advice generating device 30.
Fig. 11 illustrates one example of the management information generated by the advice generating device 30.
Fig. 12 illustrates one example of the management information generated by the advice generating device 30.
Fig. 13 illustrates one example of the management information generated by the advice generating device 30.
Fig. 14 illustrates one example of management information generated by a control section 32.
Fig. 15 is a view schematically illustrating an example of a configuration of a communication system in accordance with Embodiment 3 of the present invention.
Fig. 16 is a flowchart illustrating a method in accordance with Embodiment 3 of the present invention for generating an advice.
Fig. 17 is a view schematically illustrating an example of a configuration of a communication system in accordance with Embodiment 4 of the present invention.
Fig. 18 is a flowchart illustrating a method in accordance with Embodiment 4 of the present invention for generating an advice.

### Description of Embodiments

### Embodiment 1

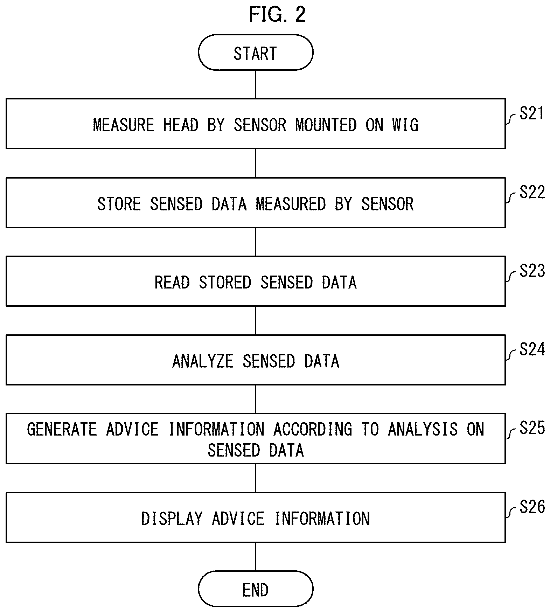

An embodiment of the present invention will be described in detail below.
(a) of Fig. 1 is a block diagram schematically illustrating a configuration of a sensor system 1 mounted on a wig in accordance with Embodiment 1 of the present invention. (b) of Fig. 1 is a block diagram schematically illustrating a configuration of an advice generating device 30 for generating advice information in accordance with sensed data obtained by measurement carried out by the sensor system 1. Fig. 2 is a flowchart illustrating a method in accordance with Embodiment 1 of the present invention for generating an advice.

### (Configuration of sensor system)

The sensor system 1 includes at least one sensor 2, a measurement section 3 for carrying out measurement on sensed data obtained by the sensor 2, and an output section 4 for controlling a device based on the data thus measured. Preferably, the sensor system 1 further includes a power source section 5.

The measurement section 3 includes a storage section 31 in which sensed data obtained by the sensor 2 is stored, a control section 32 configured to generates a signal for controlling the device based on the sensed data obtained by the sensor 2, and a timer section 33 configured to obtain a time at which the sensor 2 obtained the sensed data.

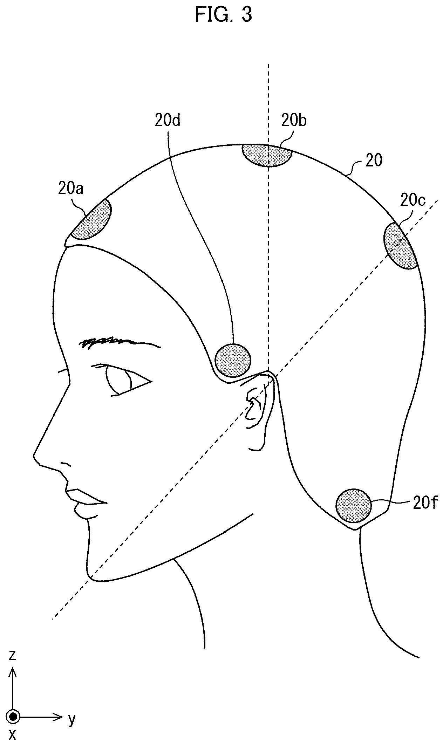

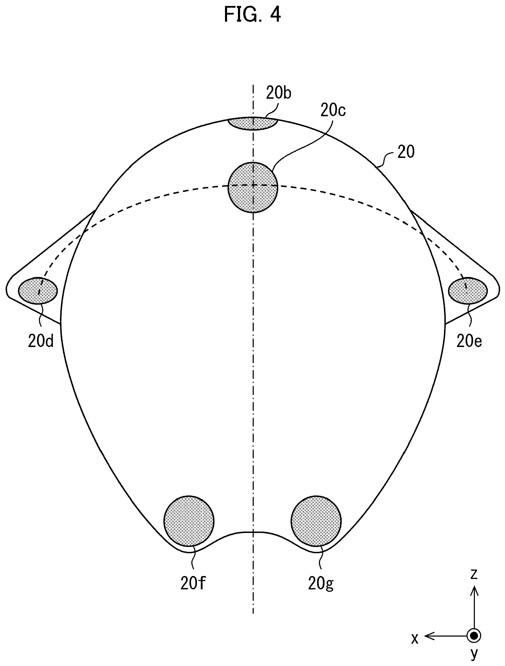

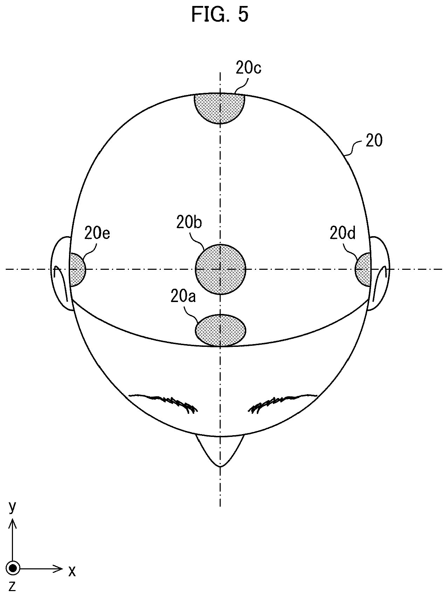

Fig. 3 is a side view schematically illustrating one example of a situation in which a wig base 20 for a full head wig is worn by a user. Fig. 4 is a back view schematically illustrating the wig base 20 for the full head wig. Fig. 5 is a top view schematically illustrating one example of a situation in which the wig base 20 for the full head wig is worn by the user. For convenience, Figs. 3 to 5 are each illustrated in a three-dimensional xyz space. Fig. 3 shows a y-z plane, Fig. 4 shows an x-z plane, and Fig. 5 shows an x-y plane.

The wig base 20 shown in Figs. 3 to 5 is one example of a wig base for a full head wig that entirely covers a head. Alternatively, the wig base 20 may be a partial wig base that partially covers a head. In Figs. 3 to 5, the wig base 20 is schematically shown as an integrated-type wig base. Alternatively, the wig base 20 may be a wig base constituted by a plurality of parts. Preferably, the wig base 20 shown in Figs. 3 to 5 is partially or entirely made of a strechable net or an unstrechable net. Although not shown in Figs. 3 to 5, hair may be implanted to the outer surface of the wig base 20 so that the wig base 20 can be used as a wig.

In an arbitrary region of the wig base 20, the sensor 2 can be mounted. In a preferable embodiment, the sensor 2 can be mounted in any of the regions 20a to 20g shown in Figs. 3 to 5. With the configuration in which the sensor 2 is directly attached to the wig base 20, it is possible to always obtain data of the same region(s) while the wig is fitted to a user's head. In a case of wearing a wig, a user intentionally fits the wig to the same site, differently from a case of wearing other accessories. Thus, with the above configuration, it is possible to always carry out sensing at the same site more accurately than wearable devices applied to other accessories.

The constituent element(s) of the sensor system 1 other than the sensor 2 can also be mounted in an arbitrary region(s) of the wig base 20. In a preferable embodiment, the constituent element(s) of the sensor system 1 other than the sensor 2 can also be mounted in the regions 20a to 20g shown in Figs. 3 to 5, similarly to the sensor 2.

More specifically, the wig base 20 includes at least one sensor 2 provided in at least one of a frontal part 20a, a parietal part 20b, temporal parts 20d and 20e, nape parts 20f and 20g, and a golden point 20c. The frontal part 20a and parietal part 20b are positioned on a center line (a straight line parallel with the z-axis) passing through the nose and glabella of a wig wearer when viewed in a left-right direction (x-axis direction) of the head. The intersection of the center line (the straight line parallel with the z-axis) and a line passing through the wig wearer's chin and upper parts of the wig wearer's left and right ears is referred to as the golden point 20c.

With reference to the flowchart shown in Fig. 2, the following will describe details of the steps. In step S21, the measurement section 3 carries out measurement on the head wearing the wig with use of the sensor 2 positioned in an arbitrary location of the wig base 20. The following will describe a specific configuration of the sensor 2.

### (Sensor)

The sensor 2 is preferably at least one of a biological information sensor and an environmental information sensor. The biological information sensor is preferably at least one of a temperature measurement sensor, a sebum measurement sensor, a humidity measurement sensor, a skin moisture content measurement sensor, a pH measurement sensor, a static electricity measurement sensor, an odor sensor, and a bloodflow volume sensor. The environmental information sensor is preferably at least one of a position information obtaining sensor and a photosensor.

### (Temperature measurement sensor)

The temperature measurement sensor is not limited to a contact type temperature sensor for directly measuring the temperature of a scalp. Alternatively, the temperature measurement sensor may be a non-contact type temperature sensor. The temperature measurement sensor is preferably a sensor capable of measuring a temperature with low power consumption, such as a temperature sensor utilizing the Seebeck effect. The temperature measurement sensor is not limited to an electric sensor, but may be an optical sensor. A subject to be measured is not limited to the temperature of a scalp. Alternatively, the temperature measurement sensor can measure a wearing environment temperature at a location between the wig and the scalp or an external environment temperature at a location outside the wig.

In a preferable embodiment, temperature sensors can be positioned in a plurality of regions, such as the regions 20a to 20g, and a temperature difference between the measurement sites can be measured. For a site where the temperature information obtained therein is below a preset reference value or a site where a temperature is remarkably lower than those of the other sites, poor blood circulation is presumed. This can be basic data used to generate advice information that promotes caring for the low-temperature site. In another preferable embodiment, sensed data obtained by the temperature sensor may be used to calculate the tendency of poor blood circulation, so that the temperature sensor can be used as a bloodflow volume sensor.

### (Sebum measurement sensor)

The sebum measurement sensor is not limited to a sensor of type for measuring electric characteristics of the scalp. Alternatively, the sebum measurement sensor may be a sensor of type for measuring optical characteristics thereof. Preferably, the sebum measurement sensor is a sensor for carrying out measurement utilizing a change in transmittance and/or a change in refractive index, each of which corresponds to the amount of sebum in a certain region. It is not always necessary to measure the absolute value of the amount of sebum. Alternatively, pieces of data indicative of relative values, such as pieces of data indicative of changes over time in the respective measurement sites, may be collected.

In a case where the amount of sebum is small, dryness of the scalp is presumed. Meanwhile, in a case where the amount of sebum is large, a scalp trouble is presumed. Thus, information indicating the amount of sebum can be basic data used to generate advice information for various kinds of troubles.

### (Skin moisture content measurement sensor, humidity measurement sensor)

The skin moisture content measurement sensor and the humidity measurement sensor can measure not only a skin moisture content of the scalp but also a wearing environment humidity at a location between the wig and the scalp or an external environment humidity at a location outside the wig. Particularly, the wearing environment humidity measured at a location between the wig and the scalp can be basic data used to numerically express the degree of stuffiness. It is preferable to sense the skin moisture content by measuring an impedance, a conductivity of high-frequency electricity, or an electrostatic capacity with use of electrodes brought into contact with the scalp. Particularly, in a case where the skin moisture content is to be sensed by measurement of an electric capacity, the measurement is preferably carried out through measurement of the amount of change in high-frequency conductance so that the measurement can be carried out with high accuracy. In a case where the moisture content is large, an effect of the moisture is presumed. Meanwhile, in a case where the moisture content is small, a trouble caused by dryness is presumed. Thus, information indicating the skin moisture content can be basic data used to generate advice information for various kinds of troubles.

### (pH measurement sensor)

The pH measurement sensor is not limited to the one employing a method of measuring an electric potential difference with use of electrodes to measure a hydrogen ion concentration. Alternatively, the pH measurement sensor may be a semiconductor sensor such as an ion sensitive field effect transistor (ISFET). From the pH sensed data obtained as a result of pH measurement carried out on the scalp, it is possible to grasp an alkaline inclination of the scalp and to presume deterioration of the sebum. Thus, the pH sensed data can be basic data used to generate advice information to advise to reconsider the shampoo.

### (Static electricity measurement sensor)

Preferably, the static electricity measurement sensor measures not only the surface potential of the scalp but also the surface potential of the wig. The surface potential is preferably measured in a contactless manner. However, in order to carry out the measurement and removal of static electricity at the same time, the surface potential can be measured in a contact manner. From the amount of generated static electricity, an effect of dryness of the scalp is presumed. The information indicative of the amount of static electricity can be basic data used to generate advice information for various kinds of troubles.

### (Odor sensor)

The odor sensor is preferably a semiconductor sensor, such as an oxide semiconductor sensor (e.g., a metal oxide semiconductor) or an organic semiconductor sensor. Depending on the odor degree of the user, it is possible to use another type of sensor, such as a membrane-type surface stress sensor (MSS) or FET biosensor. From the amount of generated odor, discomfort caused by odor is presumed. Thus, the information indicative of the amount of odor can be basic data used to generate advice information for various kinds of troubles.

### (Other biological information sensors)

Thus, various specific examples of the biological information sensor have been indicated above. However, the biological information sensor that can be employed in the present invention are not limited to the above-described sensors. For example, a sensor for sensing the viscoelasticity (tension) and/or rigidity of the scalp may be employed. Specifically, it is possible to carry out impedance measurement with use of an oscillator-incorporated device, such as MEMS.

### (Position information obtaining sensor)

As an environmental information sensor, the position information obtaining sensor may be mounted in an arbitrary region of the wig base 20. In a case where the user of the wig also uses a portable information terminal including a navigation satellite system, it is preferable to use the navigation satellite system of the portable information terminal. In this case, a first communication section 6 (described later), which is communicable with the portable information terminal with low electric power, corresponds to the position information obtaining sensor. The communication with low electric power can be achieved by Bluetooth (registered trademark) Low Energy (BLE), ZigBee (registered trademark) (IEEE 802.15.4), Ultra-WideBand (UWB), or the like.

In another preferable embodiment, the navigation satellite system of the portable information terminal is not used, and the wig base 20 includes a position information obtaining sensor. In this case, the position information obtaining sensor is not limited to global positioning system (GPS), and is preferably a sensor that can use Gallileo, GLONASS, Compass, or the like.

Preferably, the sensed data obtained by the above-described biological information sensor and position information indicative of the position where the sensed data was obtained are stored in the storage section 31 in association with each other.

### (Photosensor)

The photosensor only needs to be a sensor capable of converting the detected light quantity into a signal, and is preferably operable with a low electric power. The photosensor is preferably a sensor including a semiconductor element that can detect at least light having a wavelength within a visible region.

The photosensor may be an image capturing element. In a case where the photosensor that is an image capturing element is mounted on the wig base 20, it is possible to use the image capturing element as the sebum measurement sensor, the skin moisture content measurement sensor, or the bloodflow volume sensor, each of which is described above. By making a comparison between plural pieces of information of images captured by the image capturing element, it is possible to measure a relative value(s) of the amount of sebum, the skin moisture content, or the bloodflow volume.

### (Power source)

The power source 5 is preferably mounted in an arbitrary region of the wig base 20, similarly to the sensor 2. In a preferable embodiment, the power source 5 is preferably mounted in the region 20f or the region 20g of the wig base 20. The sensor 2 needs to be directly mounted on the wig base 20 so that the sensor 2 can sense information about the head. However, in a case where the power source 5 is a high-output power source, the sensor 2 may be mounted at a location outside the wig base 20 via a cable or the like.

The power source 5 may be a non-rechargeable battery. Preferably, however, the power source 5 is a rechargeable battery. The power source 5 is preferably configured to be rechargeable periodically during a period while the user does not wear the wig.

In another preferable embodiment, the power source 5 is preferably a self-powered thermoelement. For example, the power source 5 may be a thermoelement which is made of a silicon-based semiconductor utilizing the Seebeck effect and which is configured to generate electric power by utilizing a temperature difference between a body temperature and an atmospheric temperature.

The power source 5 may be a power source made of a combination of the above-described rechargeable battery and the above-described self-powered thermoelement.

The sensor 2 and the storage section 31 are powered by the power source 5, and consequently sensed data is obtained and stored. The timer section 33 is powered by the power source 5, and consequently a sensed time at which the sensor 2 carried out the sensing can be stored, too. Thus, the continuous and stable supply of the electric power makes it possible to obtain, over time, head environment data.

### (Reading of sensed data)

In step S22 in Fig. 2, the measurement section 3 stores, in the storage section 31, the sensed data obtained by the above-described sensor 2. The storage section 31 in which the sensed data obtained by the sensor 2 is stored is preferably a storage device detachable from the wig base 20. In a preferable embodiment, the storage device is a computer-readable storage medium constituted by a semiconductor memory such as a flash memory.

In step S23 in Fig. 2, the sensed data stored in the storage section 31 is read. In a preferable embodiment, the sensed data is read by the advice generating device 30. In the configuration example shown in (b) of Fig. 1, the advice generating device 30 includes a reading section 34, a control section 35, a storage section 38, and a display section 39. The control section 35 further includes an analyzing section 36 and an advice generating section 37. In a preferable embodiment, the reading section 34 can read the sensed data (S23). It is preferable that the sensed data read by the reading section 34 be sequentially stored in the storage section 38.

### (Generation of advice)

Next, the sensed data thus read is analyzed by the analyzing section 36 (S24), and advice information is generated by the advice generating section 37 in accordance with the analysis on the sensed data (S25). Then, the control section 35 causes the display section 39 to display the advice information thus generated (S26). Then, the process of the flowchart shown in Fig. 2 ends.

The storage section 38 may include a database. With reference to the database, the advice generating section 37 can generate the advice information in accordance with the sensed data. Here, the advice information may be data indicating advice itself (e.g., text data or image data) or data for making a selection from among plural candidate advices prepared in advance.

In a case where the advice generating device 30 is constituted by a personal computer located in a shop, advice information for displaying a large volume of text data may be adopted. With this, it is possible to display a detailed advice. Meanwhile, in a case where the advice generating device 30 is constituted by a portable information terminal such as a tablet computer or a smartphone, advice information indicating an image such as an icon may be adopted. With this, it is possible to display a certain advice.

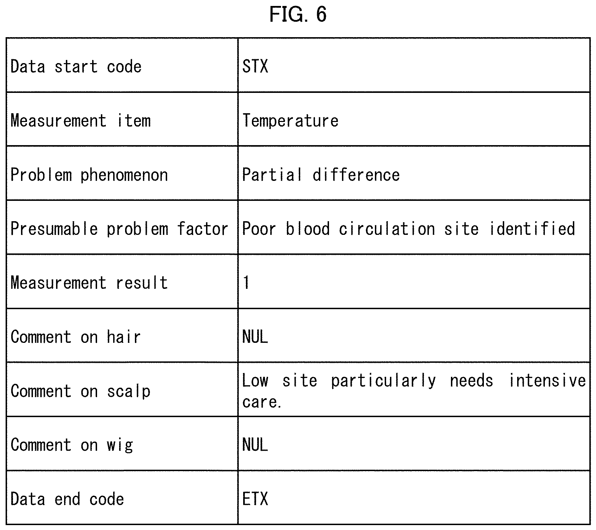

Figs. 6 to 13 are views each showing one example of a data format that defines advice information generated by the advice generating device 30. The data formats shown in Figs. 6 to 13 are examples of a variable-length text format. In another preferable embodiment, the date format may be constituted by a binary format.

### (Advice in accordance with temperature measurement data)

Fig. 6 shows one example of the management information generated by the advice generating device 30 in a case where the sensor 2 is a plurality of temperature measurement sensors and sensed data including different pieces of temperature data obtained in the plurality of regions 20a to 20g is taken into the advice generating device 30. As one example, the management information shown in Fig. 6 is managed as a variable-length text format by the advice generating device 30.

In a case where the analyzing section 36 determines, based on the sensed data, that the subject to be measured is a temperature, the analyzing section 36 enters "temperature" in a "measurement item" field in the management information, as shown in Fig. 6. If there exists a measurement result, the analyzing section 36 enters "1" in a "measurement result" field. Here, the measurement result field with "1" indicates that a measurement result exists.

In a case where the analyzing section 36 determines, based on the sensed data, that a problem phenomenon is a "partial difference", the analyzing section 36 enters "poor blood circulation site identified" for a "presumable problem factor". In accordance with the analysis information given by the analyzing section 36, the advice generating section 37 can generate, as an advice on the scalp, an advice notifying that a partial difference in scalp temperature exists. For example, the advice generating section 37 generates, as a comment on the scalp, an advice stating the following: "Low-temperature site particularly needs intensive care." In a preferable embodiment, the advice generating section 37 sends the data format shown in Fig. 6 to the display section 39.

### (Advice in accordance with sebum measurement data)

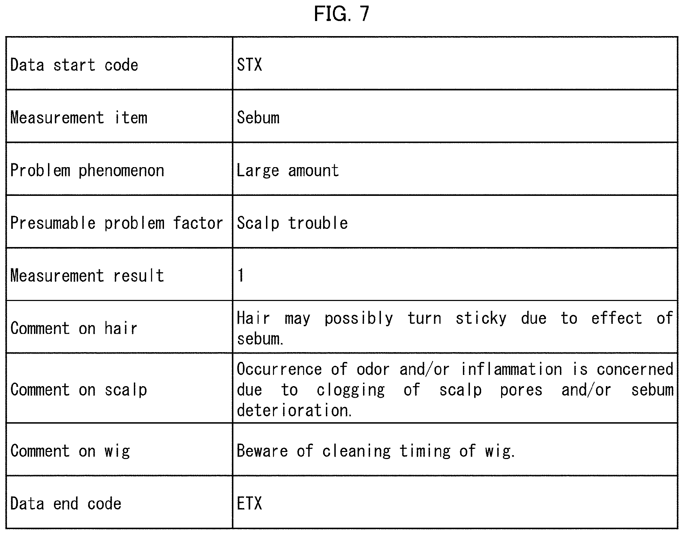

Fig. 7 shows one example of the management information generated by the advice generating device 30 in a case where the sensor 2 is a sebum measurement sensor and sensed data which is obtained by the sebum measurement sensor and which includes data indicating that the amount of sebum is large is taken into the advice generating device 30. As one example, the management information shown in Fig. 7 is managed as a variable-length text format by the advice generating device 30.

In a case where the analyzing section 36 determines, based on the sensed data, that the subject to be measured is the amount of sebum, the analyzing section 36 enters "sebum" in a "measurement item" field in the management information, as shown in Fig. 7. If there exists a measurement result, the analyzing section 36 enters "1" in a "measurement result" field. Here, the measurement result field with "1" indicates that a measurement result exists.

In a case where the analyzing section 36 determines, based on the sensed data, that a problem phenomenon is a "large amount", the analyzing section 36 enters "scalp trouble" for a "presumable problem factor". In accordance with the analysis information given by the analyzing section 36, the advice generating section 37 can generate an advice notifying that a trouble may possibly occur in a wig wearing environment due to a large amount of sebum. For example, the advice generating section 37 generates, as a comment on hair, an advice stating the following: "Hair may possibly turn sticky due to effect of sebum." Further, the advice generating section 37 generates, as a comment on the scalp, an advice stating the following: "Occurrence of odor and/or inflammation is concerned due to clogging of scalp pores and/or sebum deterioration." In addition, the advice generating section 37 generates, as a comment on the wig, an advice stating the following: "Beware of cleaning timing of wig." In a preferable embodiment, the advice generating section 37 sends the data format shown in Fig. 7 to the display section 39.

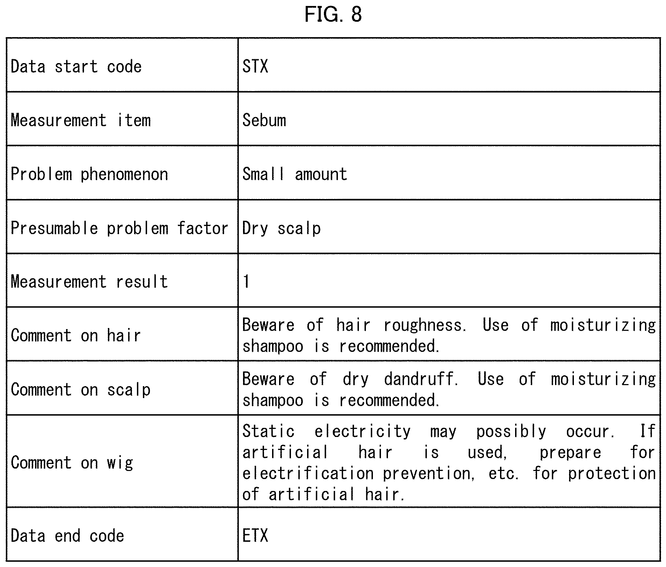

Fig. 8 shows one example of the management information generated by the advice generating device 30 in a case where the sensor 2 is a sebum measurement sensor and sensed data which is obtained by the sebum measurement sensor and which includes data indicating that the amount of sebum is small is taken into the advice generating device 30. As one example, the management information shown in Fig. 8 is managed as a variable-length text format by the advice generating device 30. In a case where the analyzing section 36 determines, based on the sensed data, that the subject to be measured is the amount of sebum, the analyzing section 36 enters "sebum" in a "measurement item" field in the management information, as shown in Fig. 8. If there exists a measurement result, the analyzing section 36 enters "1" in a "measurement result" field. Similarly to the case shown in Fig. 7, the measurement result field with "1" indicates that a measurement result exists.

In a case where the analyzing section 36 determines, based on the sensed data, that a problem phenomenon is a "small amount", the analyzing section 36 enters "dry scalp" for a "presumable problem factor". In accordance with the analysis information given by the analyzing section 36, the advice generating section 37 can generate an advice notifying that a trouble may possibly occur in a wig wearing environment due to shortage of sebum. For example, the advice generating section 37 generates, as a comment on hair, an advice stating the following: "Beware of hair roughness. Use of moisturizing shampoo is recommended." Further, the advice generating section 37 generates, as a comment on the scalp, an advice stating the following: "Beware of dry dandruff. Use of moisturizing shampoo is recommended." In addition, the advice generating section 37 generates, as a comment on the wig, an advice notifying as follows: "Static electricity may possibly occur. If artificial hair is used, prepare for electrification prevention, etc. for protection of artificial hair." In a preferable embodiment, the advice generating section 37 sends the data format shown in Fig. 8 to the display section 39.

### (Advice in accordance with humidity measurement data)

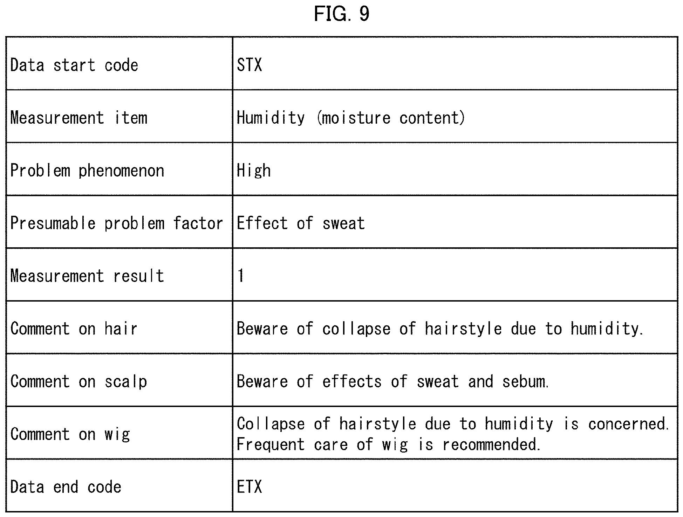

Fig. 9 shows one example of the management information generated by the advice generating device 30 in a case where the sensor 2 is a humidity measurement sensor and sensed data which is obtained by the humidity measurement sensor and which includes data indicating that the moisture content is large is taken into the advice generating device 30. As one example, the management information shown in Fig. 9 is managed as a variable-length text format by the advice generating device 30.

In a case where the analyzing section 36 determines, based on the sensed data, that the subject to be measured is humidity (moisture content), the analyzing section 36 enters "humidity (moisture content)" in a "measurement item" field in the management information, as shown in Fig. 9. If there exists a measurement result, the analyzing section 36 enters "1" in a "measurement result" field. Here, the measurement result field with "1" indicates that a measurement result exists.

In a case where the analyzing section 36 determines, based on the sensed data, that a problem phenomenon is a "high", the analyzing section 36 enters "effect of sweat" for a "presumable problem factor". In accordance with the analysis information given by the analyzing section 36, the advice generating section 37 can generate an advice notifying that a trouble may possibly occur in a wig wearing environment due to a large moisture content. For example, the advice generating section 37 generates, as a comment on hair, an advice stating the following: "Beware of collapse of hairstyle due to humidity." Further, the advice generating section 37 generates, as a comment on the scalp, an advice stating the following: "Beware of effects of sweat and sebum." In addition, the advice generating section 37 generates, as a comment on the wig, an advice stating the following: "Collapse of hairstyle due to humidity is concerned. Frequent care of wig is recommended." In a preferable embodiment, the advice generating section 37 sends the data format shown in Fig. 9 to the display section 39.

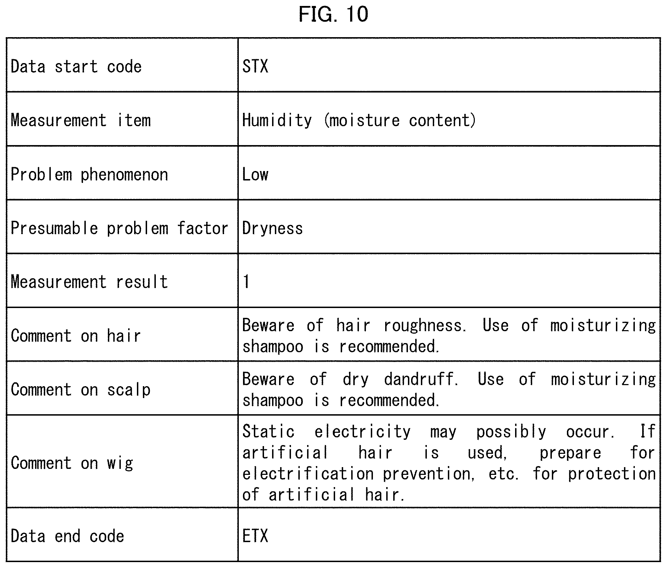

Fig. 10 shows one example of the management information generated by the advice generating device 30 in a case where the sensor 2 is a humidity measurement sensor and sensed data which is obtained by the humidity measurement sensor and which includes data indicating that the moisture content is small is taken into the advice generating device 30. As one example, the management information shown in Fig. 10 is managed as a variable-length text format by the advice generating device 30. In a case where the analyzing section 36 determines, based on the sensed data, that the subject to be measured is humidity (moisture content), the analyzing section 36 enters "humidity (moisture content)" in a "measurement item" field in the management information, as shown in Fig. 10. If there exists a measurement result, the analyzing section 36 enters "1" in a "measurement result" field. Similarly to the case shown in Fig. 9, the measurement result field with "1" indicates that a measurement result exists.

In a case where the analyzing section 36 determines, based on the sensed data, that a problem phenomenon is a "low", the analyzing section 36 enters "dryness" for a "presumable problem factor". In accordance with the analysis information given by the analyzing section 36, the advice generating section 37 can generate an advice notifying that a trouble may possibly occur in a wig wearing environment due to moisture shortage. For example, the advice generating section 37 generates, as a comment on hair, an advice stating the following: "Beware of hair roughness. Use of moisturizing shampoo is recommended." Further, the advice generating section 37 generates, as a comment on the scalp, an advice stating the following: "Beware of dry dandruff. Use of moisturizing shampoo is recommended." In addition, the advice generating section 37 generates, as a comment on the wig, an advice notifying as follows: "Static electricity may possibly occur. If artificial hair is used, prepare for electrification prevention, etc. for protection of artificial hair." In a preferable embodiment, the advice generating section 37 sends the data format shown in Fig. 10 to the display section 39.

### (Advice in accordance with pH measurement data)

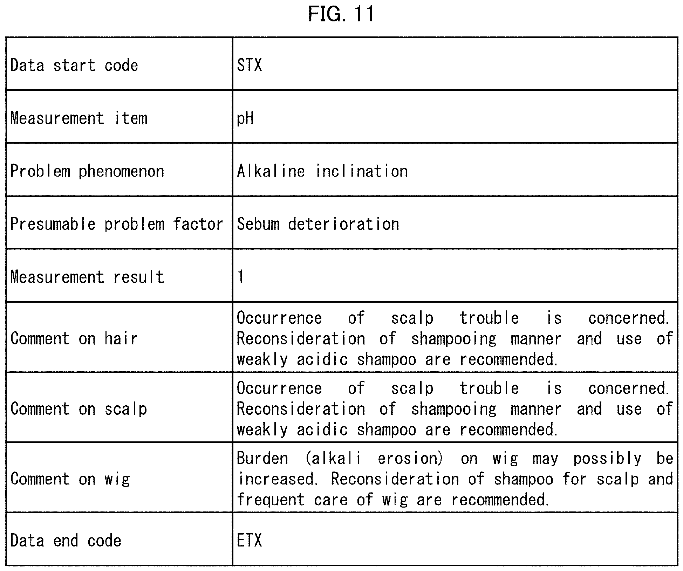

Fig. 11 shows one example of the management information generated by the advice generating device 30 in a case where the sensor 2 is a pH measurement sensor and sensed data which is obtained by the pH measurement sensor and which includes data indicating an alkaline inclination is taken into the advice generating device 30. As one example, the management information shown in Fig. 11 is managed as a variable-length text format by the advice generating device 30. In a case where the analyzing section 36 determines, based on the sensed data, that the subject to be measured is pH, the analyzing section 36 enters "pH" in a "measurement item" field in the management information, as shown in Fig. 11. If there exists a measurement result, the analyzing section 36 enters "1" in a "measurement result" field. Here, the measurement result field with "1" indicates that a measurement result exists.

In a case where the analyzing section 36 determines, based on the sensed data, that a problem phenomenon is an "alkaline inclination", the analyzing section 36 enters "sebum deterioration" for a "presumable problem factor". In accordance with the analysis information given by the analyzing section 36, the advice generating section 37 can generate an advice notifying that a trouble may possibly occur in a wig wearing environment due to alkali. For example, the advice generating section 37 generates, as comments on hair and scalp, an advice stating the following: "Occurrence of scalp trouble is concerned. Reconsideration of shampooing manner and use of weakly acidic shampoo are recommended." In addition, the advice generating section 37 generates, as a comment on the wig, an advice notifying as follows: "Burden (alkali erosion) on wig may possibly be increased. Reconsideration of shampoo for scalp and frequent care of wig are recommended." In a preferable embodiment, the advice generating section 37 sends the data format shown in Fig. 11 to the display section 39.

### (Advice in accordance with static electricity measurement data)

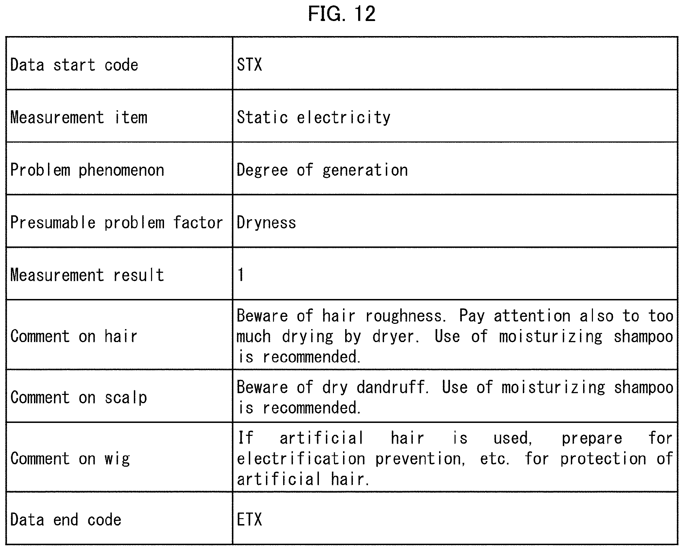

Fig. 12 shows one example of the management information generated by the advice generating device 30 in a case where the sensor 2 is a static electricity measurement sensor and sensed data which is obtained by the static electricity measurement sensor and which includes data indicating the degree of static electricity generated is taken into the advice generating device 30. As one example, the management information shown in Fig. 12 is managed as a variable-length text format by the advice generating device 30. In a case where the analyzing section 36 determines, based on the sensed data, that the subject to be measured is static electricity, the analyzing section 36 enters "static electricity" in a "measurement item" field in the management information, as shown in Fig. 12. If there exists a measurement result, the analyzing section 36 enters "1" in a "measurement result" field. Here, the measurement result field with "1" indicates that a measurement result exists.

In a case where the analyzing section 36 determines, based on the sensed data, that a problem phenomenon is a "degree of generation", the analyzing section 36 enters "dryness" for a "presumable problem factor". In accordance with the analysis information given by the analyzing section 36, the advice generating section 37 can generate an advice notifying that a trouble may possibly occur in a wig wearing environment due to static electricity. For example, the advice generating section 37 generates, as a comment on hair, an advice stating the following: "Beware of hair roughness. Pay attention also to too much drying by dryer. Use of moisturizing shampoo is recommended." Further, the advice generating section 37 generates, as a comment on the scalp, an advice stating the following: "Beware of dry dandruff. Use of moisturizing shampoo is recommended." In addition, the advice generating section 37 generates, as a comment on the wig, an advice notifying as follows: "If artificial hair is used, prepare for electrification prevention, etc. for protection of artificial hair." In a preferable embodiment, the advice generating section 37 sends the data format shown in Fig. 12 to the display section 39.

### (Advice in accordance with odor measurement data)

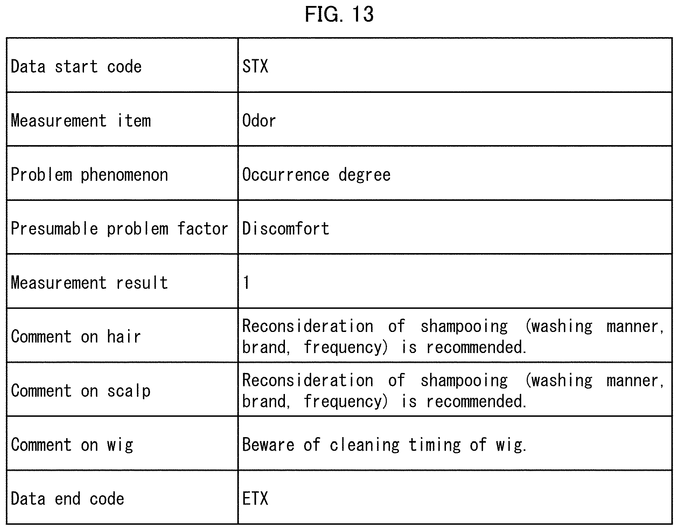

Fig. 13 shows one example of the management information generated by the advice generating device 30 in a case where the sensor 2 is an odor measurement sensor and sensed data which is obtained by the odor measurement sensor and which includes data indicating the occurrence degree of odor is taken into the advice generating device 30. As one example, the management information shown in Fig. 13 is managed as a variable-length text format by the advice generating device 30.

In a case where the analyzing section 36 determines, based on the sensed data, that the subject to be measured is odor, the analyzing section 36 enters "odor" in a "measurement item" field in the management information, as shown in Fig. 13. If there exists a measurement result, the analyzing section 36 enters "1" in a "measurement result" field. Here, the measurement result field with "1" indicates that a measurement result exists.

In a case where the analyzing section 36 determines, based on the sensed data, that a problem phenomenon is an "occurrence degree", the analyzing section 36 enters "discomfort" for a "presumable problem factor". In accordance with the analysis information given by the analyzing section 36, the advice generating section 37 can generate an advice notifying that a trouble may possibly occur in a wig wearing environment due to odor. For example, the advice generating section 37 generates, as comments on hair and scalp, an advice stating the following: "Reconsideration of shampooing (washing manner, brand, frequency) is recommended." In addition, the advice generating section 37 generates, as a comment on the wig, an advice stating the following: "Beware of cleaning timing of wig." In a preferable embodiment, the advice generating section 37 sends the data format shown in Fig. 13 to the display section 39.

### (Generation of advice involving use of image data)

In a case where the sensor 2 is an image capturing element, the image capturing element can obtain a scalp environment as image data, as described above. Based on the image data obtained as sensed data, the analyzing section 36 can analyze the amount of sebum, the skin moisture content, the bloodflow volume, and the like. Thus, the image capturing element is usable as a sebum measurement sensor, a skin moisture content measurement sensor, and a bloodflow volume sensor.

### (Generation of advice involving use of machine learning)

By specific methods for the data analysis process and the advice generation process that the analyzing section 36 and the advice generating section 37 carry out with reference to the sensed data being stored in the storage section 31 and including image data, Embodiment 1 is not limited. It is possible to adopt any one of or any combination of the following machine learning methods, for example.
- Support vector machine (SVM)
- Clustering
- Inductive logic programming (ILP)
- Genetic programming (GP)
- Bayesian network (BN)
- Neural network (NN)

In a case where the neural network is used, sensed data to be input may be processed in advance for input to the neural network. It is possible to apply, to such processing, a method such as data augmentation in addition to arranging data in a one-dimensional array or a multidimensional array.

In a case where the neural network is used, it is possible to use a convolutional neural network (CNN) including convolutional processing or a recurrent neural network (RNN) including recursive processing. In a case where the CNN is used, more specifically, the neural network can be configured to include, as one or more layers, a convolution layer(s) in (each of) which a convolution operation is performed, and to carry out a filtering operation (product-sum operation) with respect to input data which is inputted to the convolution layer(s). Meanwhile, in a case where the filtering operation is carried out, it is possible to also use processing such as padding, or to use a stride width which is appropriately set.

It is possible to use, as the neural network, a multilayer or super multilayer neural network having several tens to several thousands of layers.

The machine learning to be used for the data processing of the sensed data may be supervised leaning or unsupervised learning.

The program and/or data to be used for the data processing is/are preferably stored in the storage section 38 of the advice generating device 30, or may be stored in an external storage.

With the above-described machine learning method, the analyzing section 36 can evaluate the amount of sebum, the skin moisture content, the bloodflow volume, and/or the like.

In generation of an advice, the advice generating section 37 may use a combination of plural kinds of sensors. With this, the advice generating section 37 can generate a plurality of advices. In addition to the sensors, the advice generating section 37 may also use the analysis on the amount of sebum, the skin moisture content, and the bloodflow volume, the analysis being carried out by the analyzing section 36 with use of image data. With this, the advice generating section 37 can generate more comprehensive advice information.

### (Generation of advice by edge computing)

In the above-described examples, the advice is generated by the advice generating device 30, which is separated from the sensor system 1. However, Embodiment 1 is not limited to this.

Alternatively, the control section 35 included in the advice generating device 30 may be partially or entirely implemented by the sensor system 1, and the sensor system 1 may generate an advice on the wig. In this case, as one example, the reading section 34 reads the advice thus generated, and the display section 39 displays the advice.

### Embodiment 2

Another embodiment of the present invention will be described below. For convenience, members having functions identical to those of the respective members described in the above-described Embodiment are given respective identical reference numerals, and a description of those members is omitted here.

In Embodiment 2, the control section 32 shown in Fig. 1 preferably generates a control signal for improving a wig wearing environment in accordance with sensed data obtained by the at least one sensor 2. Various devices may be connected to the output section, which is configured to output the control signal, so that the devices can be controlled based on sensed data regarding the head wearing the wig. With this, it is possible to improve the wig wearing environment.

### (Temperature control)

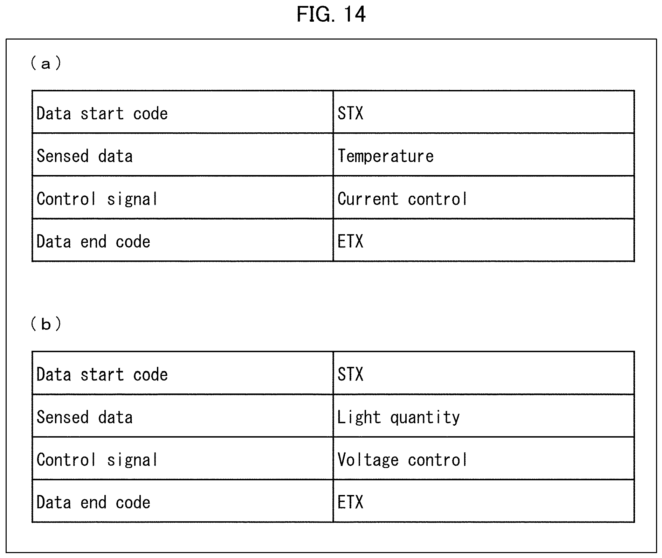

(a) of Fig. 14 shows one example of a variable-length text format generated by the control section 32 in a case where the sensor 2 is a temperature measurement sensor and the sensed data is temperature information.

In a case where a device to be controlled is a cooling element, the cooling element may be connected to the output section, which is configured to output the control signal. With this, the cooling element is included in the sensor system 1. The control section 32 causes the output section to output an output signal. In a case where at least one of the at least one sensor 2 is a temperature measurement sensor, the analyzing section 36 identifies that the sensed data obtained by the temperature measurement sensor is temperature information. Then, as shown in (a) of Fig. 14, the analyzing section 36 enters the sensed data as "temperature". In a preferable embodiment, a threshold of temperature information can be stored in the storage section 31 in advance. If the temperature information obtained by the temperature measurement sensor exceeds the preset threshold, the control section 32 generates a control signal for actuating the cooling element. In a preferable embodiment, the cooling element is a Peltier device, which is regulated by a direct current. As shown in (a) of Fig. 14, in a preferable embodiment, the control section 32 enters current control as a control signal. In accordance with the temperature information obtained, the control section 32 can regulate a direct current to be generated so as to carry out temperature control via the Peltier device.

The temperature threshold is not limited to the aspect in which a fixed value is set in advance in the storage section 31. Alternatively, it is also preferable that the cooling element feed back data indicating a change in temperature environment and the control section 32 set a more appropriate threshold by, e.g., machine learning method.

The data to be referred in the machine learning method is not limited to the temperature change information. Other than the temperature change information, information such as the sebum amount information and the skin moisture content information can be referred to so as to set an appropriate threshold. The machine learning method is preferably a method in which the above-described data processing is carried out.

### (Image-capturing control)

(b) of Fig. 14 shows one example of a variable-length text format generated by the control section 32 in a case where the sensor 2 is a photosensor and the sensed data is light quantity information.

In a case where a device to be controlled is an image capturing element, the image capturing element may be connected to the output section, which is configured to output the control signal. With this, the image capturing element is included in the sensor system 1. The control section 32 causes the output section to output an output signal. In a case where at least one of the at least one sensor 2 is a photosensor, the analyzing section 36 identifies that the sensed data obtained by the temperature measurement sensor is light quantity information. Then, as shown in (b) of Fig. 14, the analyzing section 36 enters the sensed data as "light quantity". In a preferable embodiment, a threshold of light quantity information can be stored in the storage section 31 in advance. If the light quantity information obtained by the photosensor exceeds the preset threshold, the control section 32 generates a control signal for actuating the image capturing element. In a preferable embodiment, the image capturing element is a semiconductor element controlled by a voltage. As shown in (b) of Fig. 14, in a preferable embodiment, the control section 32 enters voltage control as a control signal. In accordance with the light quantity information obtained, the control section 32 can regulate a voltage to be generated so as to control image-capturing carried out by the Peltier device.

As described above, in a preferable embodiment, it is possible to analyze the amount of sebum, the skin moisture content, the bloodflow volume, and/or the like based on the image information obtained by the image capturing element. Thus, the image capturing element is usable as a sebum measurement sensor, a skin moisture content measurement sensor, and/or a bloodflow volume sensor.

The image information obtained by the image capturing element is not limited to the aspect in which the image information is used as information for generating the above-described advice information. Alternatively, the image information can be used also as learning data used to set a threshold for controlling the cooling element. In accordance with the analysis on the image information, the advice generating section 37 generates advice information for improving the wearing environment. As one example, it is possible to carry out temperature control by the above-described cooling element based on the advice information. By controlling the image capturing element in this manner, it is possible to improve the wig wearing environment.

### (Combination with Embodiment 1)

The aspects of Embodiment 2 can be combined with the above-described aspects of Embodiment 1.

As one example, the advice information to be generated by the advice generating section 37 may incorporate therein information indicating that the wig wearing environment has been improved by the cooling element controlled based on the image information supplied from the image capturing element. In a preferable embodiment, the display section 39 may present, to the user, a report including such information.

### Embodiment 3

Another embodiment of the present invention will be described in detail below. For convenience, members having functions identical to those of the respective members described in the above-described Embodiments are given respective identical reference numerals, and a description of those members is omitted here.

### (Configuration of portable information terminal)

A sensor system 10 to be mounted on a wig in accordance with Embodiment 3 further includes a first communication section 6 for transmitting at least part of the sensed data stored in the storage section 31. The control section 32 can externally transmit sensed data regarding a head wearing the wig via the first communication section 6. Thus, it is possible to effectively utilize the storage section 31.

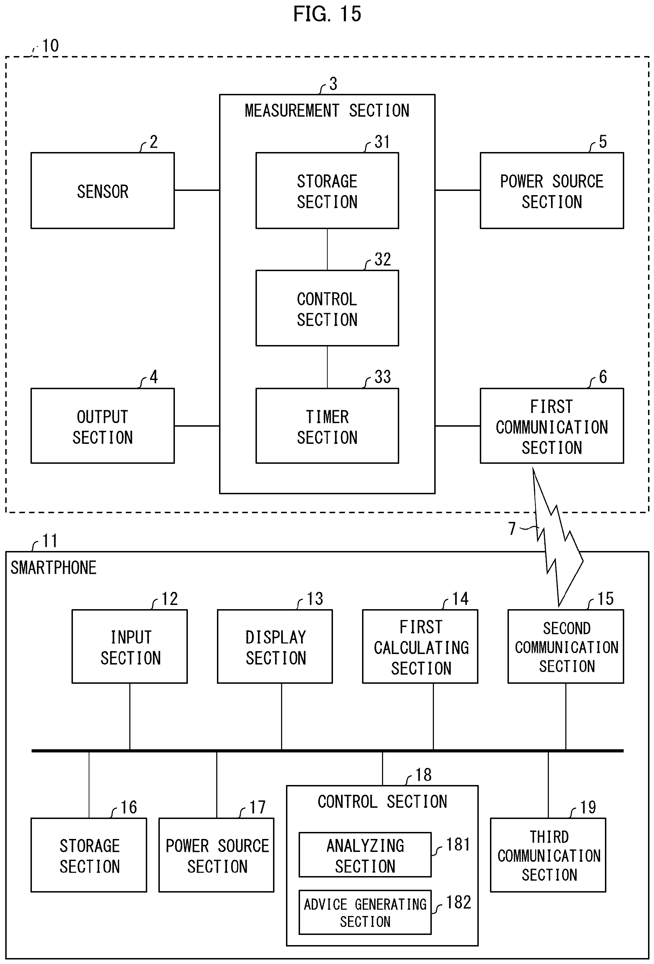

Fig. 15 schematically illustrates an example of a configuration of a communication system in accordance with Embodiment 3. Fig. 15 shows a smartphone 11, which is one example of a potable information terminal including a second communication section 15 serving as a receiver for receiving sensed data transmitted from the sensor system 10 that is a transmitter. The portable information terminal serving as the receiver is not limited to the smartphone, and may be a tablet computer or a wearable device such as a smartwatch each having a receiver function.

### (Communication with portable information terminal)

Considering that the distance between the wig worn by the user and the portable information terminal carried by the user is within a few meters, each of the first communication section 6 and the second communication section 15 is preferably a network interface compatible with the protocol of near field communication. For example, it is possible to adopt a wireless communication specification operable with a low power consumption, such as Bluetooth (registered trademark) Low Energy (BLE), ZigBee (registered trademark) (IEEE 802.15.4), or Ultra-Wide Band (UWB). However, this is not limitative.

### (Data processing in portable information terminal)

Among the constituent elements constituting the sensor system 10, at least the sensor 2 and the output section 4 are preferably mounted directly in arbitrary regions of the wig base 20. In a preferable embodiment, at least the sensor 2 and the output section 4 can be mounted in any of the regions 20a to 20g shown in Figs. 3 to 5. Similarly to the sensor 2, the first communication section 6 is preferably mounted in an arbitrary region of the wig base 20. Alternatively, the first communication section 6 may be attached at a location outside the wig base 20 via a cable and/or the like and may be retained therein.

The smartphone 11 for receiving the sensed data transmitted from the first communication section 6 includes an input section 12, a display section 13, a first calculating section 14, a second communication section 15, a storage section 16, a power source section 17, a control section 18, and a third communication section 19. The constituent elements of the smartphone 11 are operated by electric power supplied from the power source section 17.

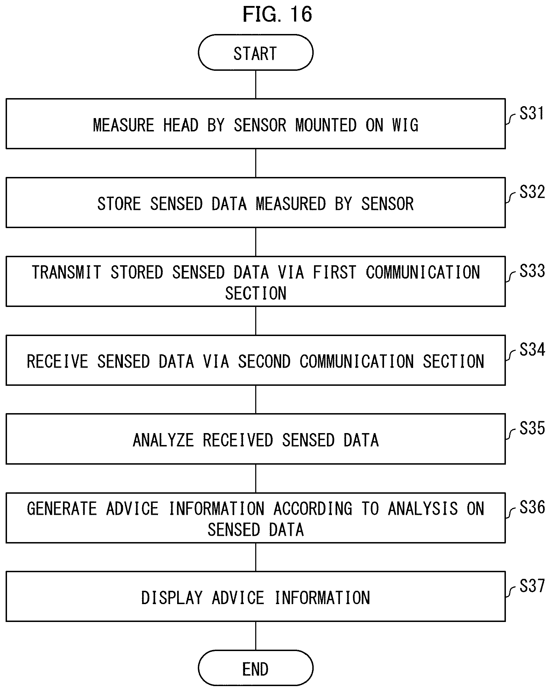

Fig. 16 is a flowchart illustrating a method in accordance with Embodiment 3 of the present invention for generating an advice.

Steps S31 and S32 in Fig. 16 are the same as Steps S21 and S22 in Fig. 2, which have been explained in Embodiment 1. In step S32, the control section 32 transmits, via the first communication section 6, the stored sensed data. The sensed data thus transmitted is received by the control section 18 of the smartphone 11 via the second communication section 15 (S33). It is preferable that the sensed data thus received be sequentially stored in the storage section 16.

An analyzing section 181 analyzes the sensed data thus received (S34), and an advice generating section 182 generates advice information in accordance with the data thus analyzed (S35). The control section 18 causes the display section 39 to display the advice information thus generated (S26). Then, the process of the flowchart shown in Fig. 16 ends.

In another preferable embodiment, a database for advice information is preferably constructed in a storage section 16. In addition, the threshold related to the temperature information and/or the threshold related to the light quantity information, each of which is described above, can be stored in the storage section 16. In order to set the threshold(s) in advance, the user can input the threshold(s) via the input section 12. The input section 12 can be a display section 13 that can function also as a touch panel.

Alternatively, the control section 18 can transmit, via the second communication section 15, the information related to the threshold(s) having been input via the input section 12, and the control section 32 can receive the information via the first communication section 6 and store the information in the storage section 31. In this case, the control section 32 of the sensor system 10 can control a control device connected to the output section 4, based on the threshold information stored in the storage section 31.

The control section 18 includes the advice generating section 182 configured to generate advice information with reference to the received sensed data. In a preferable embodiment, the analyzing section 181 analyzes the received sensed data, and the advice generating section 182 generates advice information with reference to the analyzed data and the database of the advice information. The control section 18 causes the display section 13 to display the advice information thus generated. Since the display section 13 of the smartphone 11 is provided with a screen having a limited display area, the display section 13 of the smartphone 11 preferably displays advice information as graphics information such as an icon.

In Embodiment 3, a specific example of generation of advice information is not described. The advice information may be generated by similar processes to those carried out by the analyzing section 36 and the advice generating section 37 explained in the above-described Embodiment 1.

The third communication section 19 is preferably a network interface compatible with the protocol of wireless wide area communication 22 of third or later generation communication, such as LTE communication. In a preferable embodiment, the third communication section 19 may be a network interface compatible with IEEE 802.11. However, Embodiment 3 is not limited to these communication specifications. The smartphone 11 can obtain various kinds of information via the third communication section 19. For example, the smartphone 11 can obtain weather information including weather forecast.

In a case where the sensor 2 is a position information sensor, the advice generating section 182 can generate advice information with reference to the position information of a wig wearer and the weather information. For example, in a case where rainfall forecast is obtained, the advice generating section 182 can generate advice information advising not to let the wig get wet and can cause the display section 13 to display the advice information.

### Embodiment 4

Another embodiment of the present invention will be described in detail below. For convenience, members having functions identical to those of the respective members described in the above-described Embodiments are given respective identical reference numerals, and a description of those members is omitted here.

### (Configuration of system)

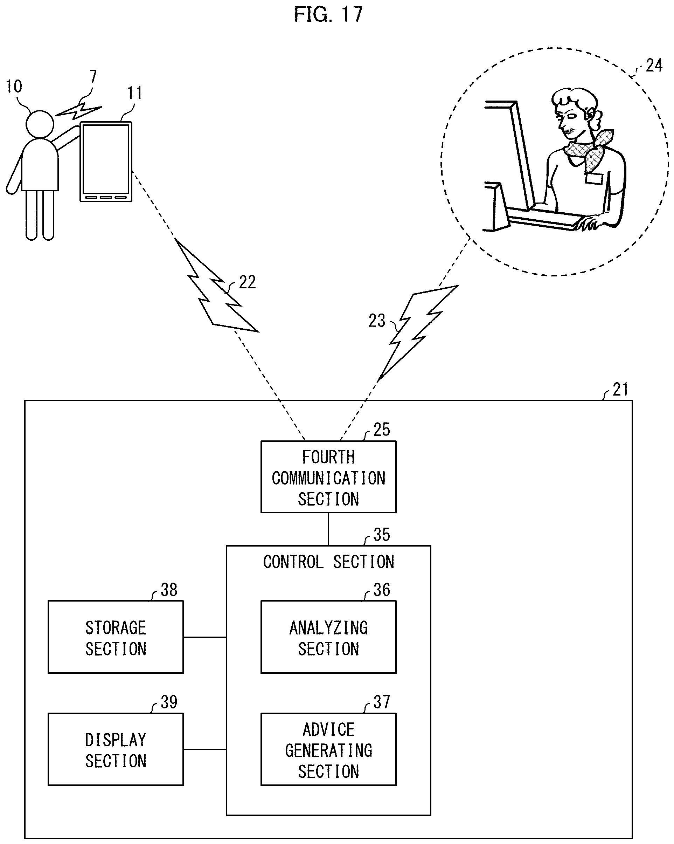

Fig. 17 schematically shows an example of a configuration of a communication system in accordance with Embodiment 4. In the example of the network configuration shown in Fig. 17, a smartphone 11 that has received sensed data transmitted from a sensor system 10 mounted on a wig base 20 is connected to a computer system 21 via wireless wide area communication 22. The computer system 21 includes at least a control section 35, a storage section 38, and a display section 39, which are in common with the advice generating device 30 of Embodiment 1. The computer system 21 further includes a fourth communication section 25. The computer system 21 preferably establishes the communication 22 with the smartphone 11 via the fourth communication section 25.

The computer system 21 can receive sensed data transmitted from a plurality of sensor systems 10 and can manage wearing environments of plural wig users. In a preferable embodiment, a manager terminal 24 can refer to the computer system 21 over a network 23.

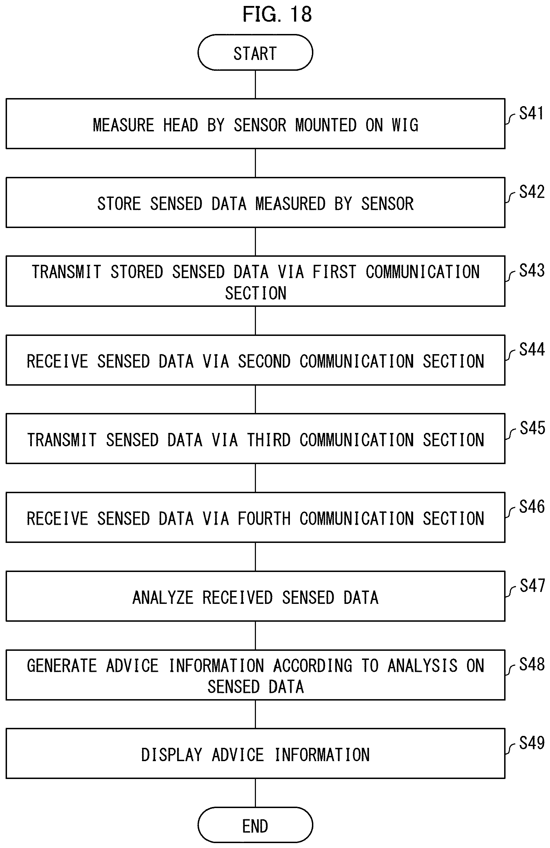

Fig. 18 is a flowchart illustrating a method in accordance with Embodiment 4 of the present invention for generating an advice.

Steps S41 and S44 in Fig. 18 are the same as Steps S31 and S34 in Fig. 16, which have been explained in Embodiment 3. In step S45, the control section 18 of the smartphone 11 transmits, via the third communication section 19, the stored sensed data. The sensed data thus transmitted is received by the control section 35 of the computer system 21 via the fourth communication section 25 (S46). It is preferable that the sensed data thus received be sequentially stored in the storage section 38 of the computer system 21.

An analyzing section 36 analyzes the sensed data thus received (S47), and an advice generating section 37 generates advice information in accordance with the data thus analyzed (S48). The control section 35 causes the display section 39 to display the advice information thus generated (S49). Then, the process of the flowchart shown in Fig. 18 ends.

In Embodiment 4, a specific example of generation of advice information is not described. The advice information may be generated by similar processes to those carried out by the analyzing section 36 and the advice generating section 37 explained in the above-described Embodiment 1.

### (Configuration of network)

In a case where the first communication section 6 is a network interface compatible with the protocol of near field communication, the sensor system 10 is preferably connected with the computer system 21 via the smartphone 11 over wireless wide area communication. Meanwhile, in a case where the first communication section 6 is a network interface compatible with Low Power Wide Area (LPWA)-network operable with low power, such as LTE Cat.M1, the sensor system 10 can be directly connected with the computer system 21 over wireless wide area communication, not via the smartphone 11.

In any of the aspects, the computer system 21 can receive sensed data transmitted from the sensor systems 10 mounted on the plurality of wig bases 20, and can store, in the database, the sensed data obtained in the various environments.

If there occurs a situation in which a certain sensor of the sensor system 10 of a certain wig user does not operate under a certain humidity environment and/or a certain temperature environment, the control section 35 can transmit information notifying it to another wig user via the fourth communication section 25.

### [Implementation example by software]

At least one of the control blocks (in particular, the measurement section 3 and the control section 35) of the sensor system 1, the control blocks (in particular, the first calculating section 14 and the control section 18) of the smartphone 11, which is an information processing device, and the control blocks (in particular, the control section 35) of the computer system 21, which is an information processing device, can be realized by a logic circuit (hardware) provided in an integrated circuit (IC chip) or the like or can alternatively be realized by software.

In the latter case, the information processing device includes a computer that executes instructions of a program that is software realizing the foregoing functions. The computer, for example, includes at least one processor and a computer-readable storage medium storing the program. An object of an aspect of the present invention can be achieved by the processor of the computer reading and executing the program stored in the storage medium. Examples of the processor encompass a central processing unit (CPU). Examples of the storage medium encompass a "non-transitory tangible medium" such as a read only memory (ROM), a tape, a disk, a card, a semiconductor memory, and a programmable logic circuit. The computer may further include a random access memory (RAM) or the like in which the program is loaded. Further, the program may be made available to the computer via any transmission medium (such as a communication network and a broadcast wave) which allows the program to be transmitted. Note that an aspect of the present invention can also be achieved in the form of a computer data signal in which the program is embodied via electronic transmission and which is embedded in a carrier wave.

Aspects of the present invention can also be expressed as follows:
A wig in accordance with a first aspect of the present invention includes: at least one sensor; and a storage section in which sensed data obtained by the at least one sensor is stored.

With the above configuration, it is possible to store, in the storage section provided to the wig, data indicative of a head environment obtained by the at least one sensor mounted on the wig.

A wig in accordance with a second aspect of the present invention can be configured to include, in the first aspect of the present invention, a power source section configured to supply power to the at least one sensor and/or the storage section, and a timer section configured to obtain a time at which the at least one sensor carries out sensing.

With the above configuration, it is possible to obtain, over time, data indicating the environment of the head wearing the wig.

A wig in accordance with a third aspect of the present invention can be configured such that, in the first or second aspect of the present invention, the at least one sensor is at least one of a biological information sensor and an environmental information sensor, the biological information sensor includes at least one of a temperature measurement sensor, a humidity measurement sensor, a pH measurement sensor, a skin moisture content measurement sensor, a sebum measurement sensor, a static electricity measurement sensor, an odor sensor, and a bloodflow volume sensor, and the environmental information sensor includes at least one of a position information obtaining sensor and a photosensor.

With the above configuration, it is possible to obtain at least one of the biological information data and the external environment data of the head wearing the wig.

A wig in accordance with a fourth aspect of the present invention can be configured to further include, in any of the first to third aspects of the present invention, a control section configured to generate a control signal for improving a wearing environment of the wig in accordance with the sensed data obtained by the at least one sensor.

With the above configuration, it is possible to generate a control signal for controlling a device configured to improve the wearing environment of the wig in accordance with the sensed data regarding the head wearing the wig.

A wig in accordance with a fifth aspect of the present invention can be configured to further include, in the fourth aspect of the present invention, at least one cooling element connected to an output section configured to output the control signal, at least one of the at least one sensor being a temperature measurement sensor, sensed data obtained by the temperature measurement sensor being temperature information, the control section being further configured to generate, in a case where the temperature information exceeds a preset threshold, a control signal for actuating the cooling element.

With the above configuration, it is possible to improve the wig wearing environment by controlling the control device based on the sensed data regarding the head wearing the wig.

A wig in accordance with a sixth aspect of the present invention can be configured to further include, in the fourth aspect of the present invention, at least one image capturing element connected to an output section configured to output the control signal, at least one of the at least one sensor being a photosensor, sensed data obtained by the photosensor being light quantity information, the control section being further configured to generate, in a case where the light quantity information exceeds a preset threshold, a control signal for actuating the image capturing element.

With the above configuration, it is possible to improve the wig wearing environment by controlling the control device based on the image information captured in accordance with the sensed data regarding the head wearing the wig.

A wig in accordance with a seventh aspect of the present invention can be configured to further include, in any of the first to sixth aspects of the present invention, a first communication section configured to transmit at least part of the sensed data stored in the storage section.

With the above configuration, it is possible to externally transmit the sensed data regarding the head wearing the wig. Thus, it is possible to effectively utilize the storage section.

An information processing device in accordance with an eighth aspect of the present invention includes: an obtaining section configured to obtain sensed data obtained by at least one sensor mounted on a wig; an advice generating section configured to generate, with reference to the sensed data obtained by the obtaining section, advice information indicating an advice for a user; and a display section configured to display the advice information generated by the advice generating section.

With the above configuration, it is possible to obtain the sensed data regarding the head wearing the wig, and to generate an advice in accordance with the obtained sensed data and display the advice.

An information processing device in accordance with a ninth aspect of the present invention can be configured such that, in the eighth aspect of the present invention, at least one of the at least one sensor is a temperature measurement sensor, sensed data obtained by the temperature measurement sensor is temperature information, and the advice generating section is further configured to generate, in a case where the temperature information is below a preset reference value, advice information that promotes caring for poor blood circulation.

With the above configuration, it is possible to obtain the temperature information regarding the head wearing the wig, and to generate an advice in accordance with the obtained temperature information and display the advice.

An information processing device in accordance with a tenth aspect of the present invention can be configured such that, in the eighth or ninth aspect of the present invention, at least one of the at least one sensor is a pH measurement sensor, sensed data obtained by the pH measurement sensor is alkaline inclination information, and the advice generating section is further configured to generate, in a case where the alkaline inclination information indicates an alkaline inclination exceeding a preset reference value, advice information that promotes reconsideration of a shampoo.

With the above configuration, it is possible to obtain the pH information regarding the head wearing the wig, and to generate an advice in accordance with the obtained pH information and display the advice.

A head measurement method in accordance with an eleventh aspect of the present invention is a method including the steps of: obtaining sensed data by carrying out, with use of at least one sensor mounted on the wig, measurement on a head wearing a wig; and storing, in a storage section mounted on the wig, the sensed data thus obtained.

With the above configuration, it is possible to store, in the storage section provided to the wig, data indicative of a head environment obtained by the at least one sensor mounted on the wig.

An information processing method in accordance with a twelfth aspect of the present invention is a method including the steps of: obtaining sensed data obtained by at least one sensor mounted on a wig; generating, with reference to the sensed data thus obtained, advice information indicating an advice for a user; and displaying the advice information thus generated.

With the above configuration, it is possible to obtain the sensed data regarding the head wearing the wig, and to generate an advice in accordance with the obtained sensed data and display the advice.

A program in accordance with a thirteenth aspect for causing a computer to function as an information processing device is a program for causing the computer to function as the information processing device of the eighth aspect, the program causing the computer to function as the obtaining section, the advice generating section, and the display section.

With the above configuration, it is possible to obtain, with use of the computer program, the sensed data regarding the head wearing the wig, and to generate an advice in accordance with the obtained sensed data and display the advice.

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

1, 10: Sensor system
2: Sensor
3: Measurement section
4: Output section
5: Power source
5, 17: Power source section
6: First communication section
11: Smartphone
12: Input section
13, 39: Display section
14: First calculating section
15: Second communication section
16, 31, 38: Storage section
18, 32, 35: Control section
19: Third communication section
20: Wig base
21: Computer system
22: Wireless wide area communication
25: Fourth communication section
30: Advice generating device
33: Timer section
34: Reading section
36, 181: Analyzing section
37, 182: Advice generating section

## Claims

1. A wig comprising:
at least one sensor; and
a storage section in which sensed data obtained by the at least one sensor is stored.

2. The wig as set forth in claim 1, further comprising:
a power source section configured to supply power to the at least one sensor and/or the storage section; and
a timer section configured to obtain a time at which the at least one sensor carries out sensing.

3. The wig as set forth in claim 1 or 2, wherein
the at least one sensor is at least one of a biological information sensor and an environmental information sensor,
the biological information sensor includes at least one of a temperature measurement sensor, a humidity measurement sensor, a pH measurement sensor, a skin moisture content measurement sensor, a sebum measurement sensor, a static electricity measurement sensor, an odor sensor, and a bloodflow volume sensor, and
the environmental information sensor includes at least one of a position information obtaining sensor and a photosensor.

4. The wig as set forth in any one of claims 1 to 3, further comprising a control section configured to generate a control signal for improving a wearing environment of the wig in accordance with the sensed data obtained by the at least one sensor.

5. The wig as set forth in claim 4, further comprising at least one cooling element connected to an output section configured to output the control signal,
at least one of the at least one sensor being a temperature measurement sensor,
sensed data obtained by the temperature measurement sensor being temperature information,
the control section being further configured to generate, in a case where the temperature information exceeds a preset threshold, a control signal for actuating the cooling element.

6. The wig as set forth in claim 4 or 5, further comprising at least one image capturing element connected to an output section configured to output the control signal,
at least one of the at least one sensor being a photosensor,
sensed data obtained by the photosensor being light quantity information,
the control section being further configured to generate, in a case where the light quantity information exceeds a preset threshold, a control signal for actuating the image capturing element.

7. The wig as set forth in any one of claims 1 to 6, further comprising a first communication section configured to transmit at least part of the sensed data stored in the storage section.

8. An information processing device comprising:
an obtaining section configured to obtain sensed data obtained by at least one sensor mounted on a wig;
an advice generating section configured to generate, with reference to the sensed data obtained by the obtaining section, advice information indicating an advice for a user; and
a display section configured to display the advice information generated by the advice generating section.

9. The information processing device as set forth in claim 8, wherein
at least one of the at least one sensor is a temperature measurement sensor,
sensed data obtained by the temperature measurement sensor is temperature information, and
the advice generating section is further configured to generate, in a case where the temperature information is below a preset reference value, advice information that promotes caring for poor blood circulation.

10. The information processing device as set forth in claim 8 or 9, wherein
at least one of the at least one sensor is a pH measurement sensor,
sensed data obtained by the pH measurement sensor is alkaline inclination information, and
the advice generating section is further configured to generate, in a case where the alkaline inclination information indicates an alkaline inclination exceeding a preset reference value, advice information that promotes reconsideration of a shampoo.

11. A head measurement method comprising the steps of:
obtaining sensed data by carrying out, with use of at least one sensor mounted on the wig, measurement on a head wearing a wig; and
storing, in a storage section mounted on the wig, the sensed data thus obtained.

12. An information processing method comprising the steps of:
obtaining sensed data obtained by at least one sensor mounted on a wig;
generating, with reference to the sensed data thus obtained, advice information indicating an advice for a user; and
displaying the advice information thus generated.

13. A program for causing a computer to function as an information processing device recited in claim 8,
the program causing the computer to function as the obtaining section, the advice generating section, and the display section.
